# EUROPÄISCHE PATENTANMELDUNG

(11) **EP 1 745 824 A1**
(43) Veröffentlichungstag der Anmeldung: **24.01.2007**
(21) Anmeldenummer: 05106586.0
(22) Anmeldetag: 19.07.2005
(51) Int. Cl.: A61Q 19/00, A61Q 17/04, A61K 8/37, A61K 8/86, A61K 8/67

(54) **Vitamin E-haltige Kosmetik-Produkte**

(71) Anmelder: Shaolin Lohan Herbals AG, 9500 Wil (CH)
(72) Erfinder: Ziegler, Jürg, 9500 Wil (CH)
(74) Vertreter: Hepp, Dieter

(57) **Zusammenfassung**

Die vorliegende Erfindung betrifft ein Kosmetik-Produkt, enthaltend Glycerylstearat, Ceteareth-20, einen polyoxyethylierten Stearylalkohol, Octylstearat und Vitamin E als essentielle Bestandteile. Es handelt sich um eine Creme, welche vorzugsweise als Massagecreme oder Gesichtscreme verwendet werden kann. Das Produkt ist sehr schonend und somit besonders für sensible Haut geeignet. Das Produkt zieht sehr schnell in die Haut ein.

## Beschreibung

Die vorliegende Erfindung betrifft neuartige Kosmetik-Produkte mit verbesserten Eigenschaften, welche insbesondere zum Auftragen auf sensible Haut geeignet sind.

Kosmetik-Produkte kommen in vielen Bereichen zur Anwendung. Beispielsweise werden derartige Produkte zur Hautpflege und als Massagecremes eingesetzt.

Es war die Aufgabe der vorliegenden Erfindung, ein Kosmetik-Produkt bereitzustellen, welches besonders zur Pflege von sensibler Haut geeignet ist und sehr schnell in die Haut einzieht.

Gemäss der vorliegenden Erfindung wird diese Aufgabe gelöst durch ein Kosmetik-Produkt, enthaltend Glycerylstearat, Ceteareth-20, einen polyoxyethylierten Stearylalkohol, Octylstearat und Vitamin E als essentielle Bestandteile.

Gemäss der vorliegenden Erfindung handelt es sich bei dem polyoxyethylierten Stearylalkohol vorzugsweise um Steareth-12 oder Steareth-20.

Gemäss einer bevorzugten Ausführungsform der vorliegenden Erfindung enthält das Kosmetik-Produkt weiterhin mindestens einen Konservierungsstoff. Vorzugsweise werden Methylparaben, Propylparaben, Iodopropylcarbamat oder ein Gemisch aus diesen Stoffen verwendet.

Die erfindungsgemässen Produkte können weitere Inhaltsstoffe enthalten, welche üblicherweise in derartigen Kosmetikprodukten eingesetzt werden. Beispielsweise können ein oder mehrere der folgenden Inhaltsstoffe eingesetzt werden: Capryltriglycerid, Cetylalkohol, PEG-75 stearat, Cetostearylalkohol, Cyclomethicon, Cetearylalkohol, Cetylpalminat, Dicaprylylcarbonat, Ascorbylmethylsilanolpectinat, Octadecendisäure, Methylsalicylat, Capsicom-Extrakt oder Bienenwachs.

Dem Produkt kann auch ein UV-Licht absorbierender Stoff zugegeben werden, beispielsweise Titandioxid.

Weiterhin kann das Produkt mindestens ein ätherisches Öl enthalten. Ätherische Öle sind bekannt und werden vom Fachmann aufgrund seines Fachwissens entsprechend dem gewünschten Anforderungsprofil ausgewählt.

Das kosmetisches Produkt enthält typischerweise ein Lösungsmittel. Vorzugsweise handelt es sich um Wasser, allein oder in Kombination mit einem organischen Lösungsmittel wie Propylenglykol.

Die genauen Konzentrationsverhältnisse der Bestandteile in den erfindungsgemässen Kosmetik-Produkten sind nicht kritisch und entsprechen den Mengen, die üblicherweise in derartigen Kosmetik-Produkten eingesetzt werden.

Bei den erfindungsgemässen Kosmetik-Produkten handelt es sich um Cremes.

Gemäss einer ersten bevorzugten Ausführungsform besteht eine erfindungsgemässe Massagecreme aus folgenden Bestandteilen: Glycerylstearat, Ceteareth-20, Steareth-20, Octylstearat, Vitamin E, Methylparaben, Propylparaben, Iodopropylcarbamat, Capryltriglycerid, Cetylalkohol, PEG-75 stearat, Cetostearylalkohol, Cyclomethicon, ätherisches Öl, Wasser und Propylenglykol.

Gemäss einer zweiten bevorzugten Ausführungsform besteht eine erfindungsgemässe Gesichtscreme aus folgenden Bestandteilen: Glycerylstearat, Ceteareth-20, Steareth-12, Octylstearat, Vitamin E, Methylparaben, Propylparaben, Iodopropylcarbamat, Cetearylalkohol, Cetylpalminat, Dicaprylylcarbonat, Ascorbylmethylsilanolpectinat, Octadecendisäure, Bienenwachs, Cyclomethicon, mikronisiertes Titandioxid, Wasser und Propylenglykol.

Gemäss einer dritten bevorzugten Ausführungsform besteht eine besonders wirksame erfindungsgemässe Massagecreme ("forte") aus folgenden Bestandteilen: Glycerylstearat, Ceteareth-20, Steareth-20, Octylstearat, Vitamin E, Methylparaben, Iodopropylcarbamat, Capryltriglycerid, Cetylalkohol, PEG-75 stearat, Cetostearylalkohol, Cyclomethicon, Methylsalicylat, Capsicom-Extrakt, Wasser und Propylenglykol.

Es hat sich gezeigt, dass diese drei Ausführungsformen besonders pflegend wirken und somit gerade für empfindliche Haut hervorragend geeignet sind. Zudem ziehen die Produkte ausserordentlich schnell ein.

Bei dem Produkt gemäss der ersten und dritten Ausführungsform handelt es sich um eine sehr schonende und feine Massagecreme, welche ausserordentlich lang einmassiert werden kann, ohne dass dies als unangenehm empfunden wird. Die Creme kann auch gegen gewisse Hautanomalien wie Schuppenflechte eingesetzt werden.

Bei dem Produkt gemäss der zweiten Ausführungsform handelt es sich um eine schonende Gesichtscreme, welche auch gegen Hautanomalien wie Akne oder Hautausschläge eingesetzt werden kann.

Die erfindungsgemässen Produkte werden in der gewünschten und üblichen Menge auf die Haut aufgetragen und gegebenenfalls einmassiert. Die Produkte ziehen sehr schnell in die Haut ein und entfalten somit sehr schnell ihre Wirkung.

Die Ausgangsstoffe für die erfindungsgemässen Produkte sind frei erhältlich. Die Herstellung der Produkte erfolgt auf herkömmliche Art durch Zusammenmischen der jeweiligen Bestandteile.

Die vorliegende Erfindung wird nachstehend anhand von nicht einschränkenden Beispielen näher erläutert.

### Beispiel 1: Massagecreme

Eine erfindungsgemässe Massagecreme wurde durch Vermischen folgender Bestandteile hergestellt:
Wasser
Capryltriglycerid
Glycerylstearat
Ceteareth-20
Steareth-20
Cetylalkohol
PEG-75 stearat
Octylstearat
Propylenglykol
Cetostearylalkohol
Cyclomethicon
Iodopropylcarbamat
Methylparaben
Propylparaben
Vitamin E
ätherisches Öl

Es wurde ein Produkt erhalten, was angenehm auf die Haut aufgetragen werden konnte und als sehr sanft empfunden wurde.

Etwa 2-3 Portionen der Massagecreme wurden auf Partien am Nacken aufgetragen und einmassiert. Die Creme fühlte sich sehr sanft an und zog sofort ein.

### Beispiel 2: Gesichtscreme

Eine erfindungsgemässe Massagecreme wurde durch Vermischen folgender Bestandteile hergestellt:
Wasser
Glycerylstearat
Ceteareth-20
Steareth-12
Cetearylalkohol
Cetylpalminat
mikronisiertes Titandioxid
Dicaprylylcarbonat
Cyclomethicon
Propylenglykol
Ascorbylmethylsilanolpectinat
Octylstearat
Bienenwachs
Octadecendisäure
Iodopropylcarbamat
Methylparaben
Propylparaben
Vitamin E

Es wurde ein Produkt erhalten, was angenehm auf die Haut aufgetragen werden konnte und als sehr sanft empfunden wurde.

Etwa 2-3 Portionen der Massagecreme wurden auf das Gesicht aufgetragen. Die Creme fühlte sich sehr sanft an und zog sofort ein.

### Beispiel 3: Massagecreme forte

Eine erfindungsgemässe Massagecreme wurde durch Vermischen folgender Bestandteile hergestellt:
Wasser
Capryltriglycerid
Glycerylstearat
Ceteareth-20
Steareth-20
Cetylalkohol
PEG-75 stearat
Octylstearat
Propylenglykol
Cetostearylalkohol
Cyclomethicon
Iodopropylcarbamat
Methylparaben
Vitamin E
Methylsalicylat
Capsicom-Extrakt

Es wurde ein Produkt erhalten, was angenehm auf die Haut aufgetragen werden konnte und als sehr sanft empfunden wurde.

Etwa 2-3 Portionen der Massagecreme wurden auf Partien am Nacken aufgetragen und einmassiert. Die Creme fühlte sich sehr sanft an und zog sofort ein und wirkte wärmend. Diese Massagecreme erwies sich als noch wirksamer als die Creme gemäss Beispiel 1.

## Patentansprüche

1. Kosmetik-Produkt, enthaltend Glycerylstearat, Ceteareth-20, einen polyoxyethylierten Stearylalkohol, Octylstearat und Vitamin E als essentielle Bestandteile.

2. Kosmetik-Produkt nach Anspruch 1, **dadurch gekennzeichnet, dass** der polyoxyethylierte Stearylalkohol ausgewählt ist aus der Gruppe bestehend aus Steareth-12 oder Steareth-20.

3. Kosmetik-Produkt nach Anspruch 1 oder 2, **dadurch gekennzeichnet, dass** weiterhin mindestens einen Konservierungsstoff, enthalten ist.

4. Kosmetik-Produkt nach einem der vorhergehenden Ansprüche, **dadurch gekennzeichnet, dass** ein oder mehrere weitere Inhaltsstoffe ausgewählt aus der Gruppe bestehend aus Capryltriglycerid, Cetylalkohol, PEG-75 stearat, Cetostearylalkohol, Cyclomethicon, Cetearylalkohol, Cetylpalminat, Dicaprylylcarbonat, Ascorbylmethylsilanolpectinat, Octadecendisäure, oder Bienenwachs enthalten sind.

5. Kosmetik-Produkt nach einem der vorhergehenden Ansprüche, **dadurch gekennzeichnet, dass** UV-Licht absorbierender Stoff, beispielsweise Titandioxid, enthalten ist.

6. Kosmetik-Produkt nach einem der vorhergehenden Ansprüche, **dadurch gekennzeichnet, dass** mindestens ein ätherisches Öl enthalten ist.

7. Kosmetik-Produkt nach einem der vorhergehenden Ansprüche, **dadurch gekennzeichnet, dass** es die folgenden Bestandteile enthält: Glycerylstearat, Ceteareth-20, Steareth-20, Octylstearat, Vitamin E, Methylparaben, Propylparaben, Iodopropylcarbamat, Capryltriglycerid, Cetylalkohol, PEG-75 stearat, Cetostearylalkohol, Cyclomethicon, ätherisches Öl, Wasser und Propylenglykol.

8. Kosmetik-Produkt nach einem der vorhergehenden Ansprüche, **dadurch gekennzeichnet, dass** es die folgenden Bestandteile enthält: Glycerylstearat, Ceteareth-20, Steareth-12, Octylstearat, Vitamin E, Methylparaben, Propylparaben, Iodopropylcarbamat, Cetearylalkohol, Cetylpalminat, Dicaprylylcarbonat, Ascorbylmethylsilanolpectinat, Octadecendisäure, Bienenwachs, Cyclomethicon, mikronisiertes Titandioxid, Wasser und Propylenglykol.

9. Kosmetik-Produkt nach einem der vorhergehenden Ansprüche, **dadurch gekennzeichnet, dass** es die folgenden Bestandteile enthält: Glycerylstearat, Ceteareth-20, Steareth-20, Octylstearat, Vitamin E, Methylparaben, Iodopropylcarbamat, Capryltriglycerid, Cetylalkohol, PEG-75 stearat, Cetostearylalkohol, Cyclomethicon, Methylsalicylat, Capsicom-Extrakt, Wasser und Propylenglykol.

10. Verwendung eines Kosmetik-Produkts gemäss Anspruch 7 oder 9 als Massagecreme.

11. Verwendung eines Kosmetik-Produkts gemäss Anspruch 8 als Gesichtscreme.
